(19) Europäisches Patentamt / European Patent Office / Office européen des brevets

(11) **EP 3 224 372 B1**

(12) **FASCICULE DE BREVET EUROPEEN**

(45) Date de publication et mention de la délivrance du brevet:
**20.03.2019 Bulletin 2019/12**

(21) Numéro de dépôt: **15808742.9**

(22) Date de dépôt: **26.11.2015**

(51) Int Cl.:
*G06T 7/20* *(2017.01)*    *G06T 7/30* *(2017.01)*
*G06T 7/33* *(2017.01)*    *C12Q 1/04* *(2006.01)*
*G06K 9/00* *(2006.01)*

(86) Numéro de dépôt international:
**PCT/FR2015/053221**

(87) Numéro de publication internationale:
**WO 2016/083744 (02.06.2016 Gazette 2016/22)**

(54) **PROCEDE, SYSTEME ET PRODUIT-PROGRAMME D'ORDINATEUR POUR DETERMINER LA CROISSANCE DE MICRO-ORGANISMES**

VERFAHREN, SYSTEM UND COMPUTERPROGRAMMPRODUKT ZUR BESTIMMUNG DES WACHSTUMS VON MIKROORGANISMEN

METHOD, SYSTEM AND COMPUTER PROGRAM PRODUCT FOR DETERMINING MICROORGANISM GROWTH

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.11.2014 FR 1461533**

(43) Date de publication de la demande:
**04.10.2017 Bulletin 2017/40**

(73) Titulaires:
• **bioMérieux**
**69280 Marcy-l'Étoile (FR)**
• **COMMISSARIAT À L'ÉNERGIE ATOMIQUE ET AUX**
**ÉNERGIES ALTERNATIVES**
**75015 Paris (FR)**

(72) Inventeurs:
• **LAGARRIGUE-CHARBONNIER, Marthe**
**75014 Paris (FR)**
• **ALLANO, Lorène**
**92310 Sèvres (FR)**
• **DEPECKER, Marine**
**91390 Morsang-sur-Orge (FR)**

(74) Mandataire: **Murgitroyd & Company**
**Scotland House**
**165-169 Scotland Street**
**Glasgow G5 8PL (GB)**

(56) Documents cités:
**EP-A1- 1 061 127        WO-A1-2014/059357**
**WO-A2-03/022999        US-A- 5 510 246**

• **ALICE L. DEN HERTOG ET AL: "Simplified Automated Image Analysis for Detection and Phenotyping of Mycobacterium tuberculosis on Porous Supports by Monitoring Growing Microcolonies", PLOS ONE, vol. 5, no. 6, 1 janvier 2010 (2010-01-01) , pages e11008-e11008, XP055012770, ISSN: 1932-6203, DOI: 10.1371/journal.pone.0011008 cité dans la demande**
• **WEI-BANG CHEN ET AL: "An automated bacterial colony counting and classification system", INFORMATION SYSTEMS FRONTIERS ; A JOURNAL OF RESEARCH AND INNOVATION, KLUWER ACADEMIC PUBLISHERS, BO, vol. 11, no. 4, 18 février 2009 (2009-02-18), pages 349-368, XP019730180, ISSN: 1572-9419, DOI: 10.1007/S10796-009-9149-0 cité dans la demande**

EP 3 224 372 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

### Domaine technique

L'invention concerne le domaine du traitement d'images produites par ordinateur, et, plus précisément, un procédé, un système et un produit-programme d'ordinateur pour déterminer la croissance de micro-organismes afin de détecter et d'identifier des micro-organismes au sein d'un objet tel qu'une boîte de Petri, après l'incubation dudit objet.

### Etat de la technique

Dans le domaine de la microbiologie, il est connu d'utiliser des images d'une boîte de Petri acquises à différents temps d'incubation afin de détecter et d'identifier d'éventuels micro-organismes. La boîte de Petri contient un milieu de culture tel qu'une gélose spécifique dans laquelle un échantillon adapté à ladite gélose est ensemencé. De manière connue, une première image de la boîte de Petri est acquise à un instant T0, préalablement à l'incubation de la boîte de Petri et au moyen d'un système d'imagerie adapté. Ainsi, la première image acquise ne comprend aucune colonie de micro-organismes. La boîte de Petri est ensuite incubée pendant une période de temps déterminée, jusqu'à un instant T1. Une deuxième image de la boîte de Petri est alors acquise à l'instant T1.

Ensuite, la première image acquise au temps T0 est comparée avec l'image acquise au temps T1, afin de déterminer si des colonies de micro-organismes sont apparues lors de la période d'incubation, c'est-à-dire si l'échantillon ensemencé a généré la croissance de colonies de micro-organismes au sein de la boîte de Petri.

Cependant, la comparaison des images de la boîte de Petri ne permet pas de détecter avec fiabilité une éventuelle présence de colonies de micro-organismes. En effet, de nombreux paramètres peuvent engendrer un résultat erroné de la comparaison des images de la boîte de Petri.

Concernant la boîte de Petri elle-même, différents paramètres peuvent engendrer une détection erronée des colonies de micro-organismes.

Un premier paramètre concerne le matériau de la boîte de Petri. Ainsi, la boîte de Petri est réalisée dans un matériau, tel que le verre ou le plastique, qui peut comporter des défauts tels que des rayures. Selon les conditions d'acquisition des images de la boîte de Petri, les rayures peuvent apparaître sur l'image de la boîte de Petri en tant qu'élément distinct. Ainsi une rayure peut être interprétée, de manière erronée, comme représentative d'une croissance de micro-organismes.

Un deuxième paramètre concerne des éléments associés à la boîte de Petri. Ainsi, la boîte de Petri comprend généralement une inscription sérigraphiée, par exemple un numéro permettant d'identifier l'origine de la production de la boîte de Petri. De manière similaire aux rayures, selon les conditions d'acquisition des images de la boîte de Petri, l'inscription sérigraphiée peut apparaître sur l'image de la boîte de Petri en tant qu'élément distinct. Ainsi une inscription sérigraphiée peut être interprétée, de manière erronée, comme représentative d'une croissance de micro-organismes.

Un troisième paramètre concerne le milieu de culture disposé au sein de la boîte de Petri, tel que la gélose. En effet, la gélose nécessaire à la mise en culture de l'échantillon peut contenir des défauts structurels. Selon les conditions d'acquisition des images de la boîte de Petri, ces défauts structurels tels que des bulles ou une rétractation de la gélose peuvent également apparaître en tant qu'éléments distincts sur l'image de la boîte de Petri. Ainsi, les défauts structurels peuvent également être interprétés, de manière erronée, comme représentatifs d'une croissance de micro-organismes.

Un quatrième paramètre concerne la présence de buée. En effet, lors de la période d'incubation, des particules d'eau formant de la buée peuvent apparaître sur les parois de la boîte de Petri. Or, lors de l'analyse de la boîte de Petri, celle-ci subit un retournement qui peut provoquer la chute des particules d'eau sur la gélose. Ainsi, selon les conditions d'acquisition d'une image de la boîte de Petri, lesdites particules d'eau peuvent apparaître sous forme d'éléments distincts sur ladite image de la boîte de Petri. Ainsi, la présence de buée peut également engendrer une interprétation erronée d'une croissance de micro-organismes.

Enfin, un cinquième paramètre concerne d'éventuels déplacements de la boîte de Petri en translation et/ou en rotation entre l'acquisition de la première image à T0 et l'acquisition de la deuxième image à T1. Ces déplacements génèrent un décalage de la position de l'objet au sein de la première image acquise par rapport à la position dudit objet au sein de la deuxième image acquise. Or, un décalage entre les images acquises aux instant T0 et T1 ne permet pas de les comparer par superposition, en vue de détecter la présence de colonies de micro-organismes.

Dans l'art antérieur, le brevet EP0796319 décrit un procédé d'identification de colonies de micro-organismes comprenant l'acquisition d'images d'un dispositif de mise en culture tel qu'une boîte de Petri ou un film dit Petrilfilm™. Le procédé comprend des étapes de recalage des images acquises en utilisant comme position de référence le centre du dispositif de mise en culture. Le recalage concerne uniquement un mouvement de translation du dispositif de mise en culture entre une première image acquise à un temps T0 et une deuxième image acquise à un temps T1. Par conséquent, le brevet EP0796319 ne décrit pas d'étape de recalage permettant de considérer un éventuel mouvement de rotation du dispositif de mise en culture entre les deux temps d'acquisition T0 et T1 des images. De plus, les conditions d'acquisition des images sont telles que les conditions d'illumination d'une image acquise au temps T0 sont identiques à celles d'une image acquise au temps T1. Ainsi, le brevet EP0796319 décrit des étapes de comparaison des images comprenant la soustraction des pixels de l'image acquise à T0 avec les pixels de l'image acquise à T1 afin d'obtenir une image de comparaison comprenant des pixels résultants dont l'intensité est éventuellement représentative d'une croissance de micro-organismes. Par conséquent, le brevet EP0796319 ne décrit pas d'étapes de comparaison d'images adaptées à des images acquises selon différentes conditions d'illumination.

Toujours dans l'art antérieur, le brevet US 5,510,246 divulgue un procédé pour déterminer la croissance de micro-organismes au sein d'un/sur un dispositif de culture substantiellement plan tel qu'une plaque de type Petrifilm™. Ce procédé comprend notamment des étapes préliminaires de filtrage d'images, plus précisément de filtrage de pixels, afin d'obtenir une image d'arrière-plan dépourvue de pixels « parasites ». Cette image d'arrière-plan est ensuite utilisée pour identifier les différences observées entre une image du dispositif de culture prise après une certaine durée d'incubation du milieu de culture (préalablement inoculé) et ladite image d'arrière-plan. Même si le brevet US 5,510,246 traite, de manière générale, d'un procédé de traitement d'images en vue de dénombrer les colonies microbiennes ayant poussé sur un milieu de culture présent au sein/sur un dispositif de culture, il est important de noter que US 5,510,246 enseigne que le procédé divulgué dans ce brevet est automatisé et considéré comme fiable en termes de positionnement du dispositif de culture, ce qui rend totalement inutile tout étape de recalage d'image, et qui plus est tout étape de recalage nécessitée par un mouvement de type rotation du dispositif de culture au cours du procédé d'acquisition d'images.

Dans l'art antérieur, la demande de brevet internationale WO 2014/059357 décrit également un procédé pour déterminer des paramètres de croissance de cellules ensemencées sur un support d'analyse tel qu'une plaque de verre. Le procédé comprend une étape de recalage des images du support d'analyse, laquelle comprend notamment l'utilisation :

- d'un algorithme connu dans l'état de l'art, l'algorithme « Starmatch », qui utilise des matrices de transformation affine ainsi que le regroupement des objets recalés dans un espace euclidien à deux dimensions, afin de déterminer la liste des points d'intérêt dans chaque image puis d'en déduire la transformation optimale, et
- d'une fonction également connue, la fonction "Gompertz", appliquée aux zones de données des objets regroupés pour déterminer les zones de croissances de micro-organismes.

Un inconvénient majeur du procédé objet de WO 2014/059357 réside dans la présence obligatoire de points d'intérêt dans une image acquise à un temps initial afin d'être en mesure de retrouver ces points d'intérêt dans une image acquise à un temps ultérieur. Par conséquent, le procédé objet de WO 2014/059357 ne permet pas de recaler des images en l'absence de colonies de micro-organismes à un temps initial.

La demande de brevet européen EP-A-1061127 divulgue, quant à elle, un procédé pour identifier des micro-organismes présents au sein d'un récipient d'analyse tel qu'une boîte de Petri. Le procédé comprend la prise de deux images, l'une après l'autre, dans l'optique de mieux déterminer les contours et les détails d'au moins une colonie de micro-organismes. Plus précisément, la première image est acquise avec une première position de la caméra et la deuxième image est acquise avec une deuxième position de la caméra. Le déplacement entre la première et la deuxième image est donc un déplacement connu (et donc prévisible), aisément identifiable sur les images. EP-A-1061127 ne divulgue, pas plus que ne suggère, d'étape de recalage d'images lors d'un déplacement imprévu/inopiné du récipient d'analyse.

En outre, l'article de DEN HERTOG, A. et al., « Simplified Automated Image Analysis for Détection and Phenotyping of Mycobacterium tubercolisis on Porous Supports by Monitoring Growing Microcolonies ». PLos ONE, (2010). Volume 5, Issue 6, e11008, décrit une méthode d'analyse de la croissance de micro-organismes au sein d'un support d'oxyde d'aluminium poreux sous forme de bandes. Le procédé permet une détection automatique des colonies de micro-organismes tout en détectant de manière spécifique les micro-colonies individuelles afin de les surveiller au cours du temps et de les identifier en fonction de leur croissance. L'article ne décrit aucune étape de recalage relative à un déplacement du support d'analyse.

Une autre publication, à savoir CHEN W-B. « An automated bacterial colony counting and classification system ». Springer Science + Business Media, (2009). Published online, Inf Syst Front, 11:349-368, décrit un procédé de comptage automatique de colonies de micro-organismes au sein d'un support d'analyse qui peut être une boîte de Petri. Le procédé comprend la détection du support d'analyse, l'identification des colonies, la séparation des colonies agrégées et la détermination du nombre de colonies. Cette publication ne divulgue aucune étape de recalage basée sur un déplacement du support d'analyse.

Le document WO 03022999 concerne une énumération rapide de cellules vivantes en détectant des colonies microscopiques dérivées de la division cellulaire in situ en utilisant une imagerie à grande surface. Des modes de réalisation de l'invention comprennent des procédés aseptiques non destructifs pour détecter des micro colonies cellulaires sans marquage de réactifs. Ces méthodes permettent la génération de cultures pures qui peuvent être utilisées pour l'identification microbienne et la détermination de la résistance aux anti-microbiens.

Par conséquent, il s'avère nécessaire d'améliorer la détection de croissance de micro-organismes au sein d'un objet tel qu'une boîte de Petri en considérant le recalage d'image lié à une translation et/ou à une rotation - pouvant se produire de manière inopinée - de la boîte de Petri lors de l'acquisition des images de la boîte de Petri à des temps différents.

**Objet de l'invention**

La présente invention vise à surmonter au moins partiellement les problèmes mentionnés ci-dessus.

Ainsi, un premier objectif de l'invention consiste à fournir un procédé pour déterminer la croissance de micro-organismes dans un échantillon biologique susceptible de contenir des micro-organismes, ledit échantillon biologique étant contenu dans un récipient d'analyse tel qu'une boîte de Petri, ledit récipient d'analyse étant soumis à une incubation d'une durée déterminée, ledit procédé comprenant les étapes suivantes :

- acquérir une première pluralité d'images initiales du récipient d'analyse à un premier temps d'acquisition T1, avant ou pendant l'incubation ;
- acquérir une deuxième pluralité d'images finales du récipient d'analyse à un deuxième temps d'acquisition T2, pendant ou après l'incubation ;
- recaler chaque image initiale de la première pluralité d'images initiales acquises, avec chaque image finale correspondante de la deuxième pluralité d'images finales acquises ;
- localiser au moins une zone potentielle de croissance de micro-organismes au sein d'au moins une image de la deuxième pluralité d'images acquises ;
- évaluer le contenu de la zone potentielle de croissance de micro-organismes identifiée afin de déterminer la présence de micro-organismes.

De manière avantageuse, l'étape de recalage comprend une étape de recalage primaire (également dénommé « recalage grossier ») associée à un identifiant localisé sur le récipient d'analyse.

De manière avantageuse, l'étape de recalage comprend une étape de recalage secondaire associée au contenu du récipient d'analyse.

Comme cela est connu de l'homme du métier, le recalage de deux images consiste à mettre en correspondance le ou les objets réels visualisés sur chacune de ces deux images. Le recalage de deux images s'avère particulièrement utile lorsqu'un déplacement du dispositif de prise d'images et/ou du ou des objets d'intérêt se produit entre l'acquisition des deux images.

De manière avantageuse, l'étape de localisation d'au moins une zone potentielle de croissance de micro-organismes comprend une étape de détection d'une densité élevée de colonies de micro-organismes, une étape de détection de colonies de micro-organismes non-circulaires et une étape d'attribution d'un élément de localisation à ladite zone potentielle de croissance de micro-organismes.

De manière avantageuse, l'étape d'évaluation du contenu de la zone potentielle de croissance identifiée comprend une étape de détermination des valeurs de deux paramètres d'évolution.

De manière avantageuse, les deux paramètres d'évolution comprennent un paramètre de corrélation et un paramètre de contraste.

De manière avantageuse, selon l'invention l'étape de recalage secondaire (également dénommé « recalage fin ») comprend la création d'un maillage de sous-images pour chaque image initiale et chaque image finale. Comme cela est connu de l'homme du métier, une « sous-image » s'entend d'une partie d'une image, de taille inférieure à celle de ladite image. Une « sous-image » permet de focaliser/centrer l'image sur un point d'intérêt ou un aspect local de l'image. La taille et/ou la position de la sous-image (au sein de l'image) est/sont variable(s) en fonction des souhaits de l'opérateur.

Le recalage secondaire permet de compenser, de manière précise, à savoir à l'échelle du pixel, le décalage (de type translation et/ou rotation) créé entre les images acquises à T1 et les images acquises à T2. Ainsi, les étapes ultérieures de localisation d'au moins une zone potentielle de croissance de micro-organismes et d'évaluation du contenu de cette zone potentielle de croissance de micro-organismes peuvent être réalisées de manière optimale, tout en limitant - voire supprimant - l'impact des artefacts (tels que la présence d'une rayure ou d'une inscription sérigraphiée sur le récipient d'analyse, par exemple sur une boîte de Petri) sur l'interprétation des résultats.

Ainsi, un deuxième objectif de l'invention consiste à fournir un système pour déterminer la croissance de micro-organismes dans un échantillon biologique susceptible de contenir des micro-organismes, ledit échantillon biologique étant contenu dans un récipient d'analyse telle qu'une boîte de Petri, ledit récipient d'analyse étant soumis à une incubation d'une durée déterminée, ledit système comprenant :

- un dispositif de capture d'images afin d'acquérir une pluralité d'images de l'objet à analyser à un premier temps d'acquisition T1, avant ou pendant l'incubation, et à un deuxième temps d'acquisition T2, pendant ou après l'incubation ;
- un dispositif d'illumination comprenant une ou plusieurs sources lumineuses afin d'illuminer le récipient d'analyse ;
- un dispositif de contrôle pour contrôler l'application du procédé de détermination de la croissance de micro-organismes selon l'invention afin de déterminer la présence de micro-organismes.

Ainsi, un troisième objectif de l'invention consiste à fournir un produit-programme d'ordinateur comprenant des instructions logicielles pour la mise en oeuvre d'un procédé selon l'invention lorsque ledit programme est exécuté par un processeur de données.

**Brève description des dessins**

L'invention, sa fonctionnalité, ses applications ainsi que ses avantages seront mieux appréhendés à la lecture de la présente description, faite en référence aux figures, dans lesquelles :

- la figure 1 montre une vue schématique d'un système d'analyse d'images selon un mode de réalisation de l'invention ;
- la figure 2 montre un diagramme des étapes du procédé global de détermination de la croissance de micro-organismes selon un mode de réalisation de l'invention ;
- la figure 3 montre un diagramme des étapes du procédé de recalage primaire selon un mode de réalisation de l'invention ;
- la figure 4 montre une image d'une boîte de Petri acquise au temps T1 ;
- la figure 5 montre une image de la boîte de Petri selon la figure 4 avec une représentation ciblée sur les pixels distants du centre de la boîte de Petri selon un rayon R-10 pixels ;
- la figure 6 montre une image de la boîte de Petri selon la figure 4 avec une représentation ciblée sur les pixels distants du centre de la boîte de Petri selon un Rayon R-50 pixels ;
- la figure 7 montre une image de la boîte de Petri selon la figure 4 en application d'une opération de segmentation ;
- les figures 8 et 9 montrent une image de la boîte de Petri selon la figure 4 après l'application d'une opération d'ouverture morphologique ;
- la figure 10 montre une image de la boîte de Petri sur la figure 4 après application d'une fonction d'érosion ;
- la figure 11 montre en détail le contenu du rectangle représenté sur la figure 10 ;
- la figure 12 montre une image de la boîte de Petri selon la figure 9 après application d'une fonction d'ouverture ;
- la figure 13 montre en détail le contenu du rectangle représenté sur la figure 12 ;
- la figure 14 montre une représentation graphique de l'étiquette latérale théorique ;
- la figure 15 montre une représentation graphique de l'étiquette latérale théorique en traits discontinus, de l'angle d'orientation θ en trait plein et de l'ellipsoïde équivalent sous forme de points ;
- la figure 16 montre une représentation graphique comparative des images de l'étiquette latérale acquise à T1 et à T2 ;
- la figure 17 montre une représentation graphique selon la figure 16 basée sur les moitiés supérieures desdites étiquettes latérales théoriques ;
- la figure 18 montre une représentation graphique des étiquettes selon la figure 16 basée sur les moitiés inférieures desdites étiquettes latérales théoriques ;
- la figure 19 montre une image d'une boîte de Petri acquise à un temps T1 ;
- la figure 20 montre une image de la boîte de Petri acquise à un temps T2 ;
- la figure 21 montre un diagramme des étapes concernant le procédé de recalage secondaire selon le mode de réalisation de l'invention ;
- la figure 22 montre la représentation d'un pixel au sein d'une cellule initiale ;
- la figure 23 montre en détail le contenu du carré représenté sur la figure 22 ;
- la figure 24 montre la représentation d'un pixel au sein d'une cellule finale ;
- la figure 25 montre en détail le contenu du carré représenté sur la figure 24 ;
- la figure 26 montre un diagramme des étapes relatives au procédé de recalage local selon la figure 21 ;
- la figure 27 montre une image de la boîte de Petri acquise à un temps T1 selon la condition d'acquisition « backlight » ;
- la figure 28 montre une image de la boîte de Petri selon la figure 27 acquise à un temps T2 selon la condition d'acquisition « backlight » ;
- la figure 29 montre en détail le contenu du carré représenté sur la figure 28 ;
- la figure 30 montre un diagramme des étapes relatives au procédé de détermination de croissance préalable de micro-organismes selon la figure 2 ;
- la figure 31 montre un diagramme des étapes du procédé d'analyse temporel ;
- la figure 32 montre un diagramme des étapes relatives au procédé de prétraitement d'images selon la figure 31 ;

- la figure 33 montre des exemples de sous-images acquise à T1 et à T2 selon les conditions d'acquisition « backlight », « bottom » et « médian » après l'application d'un procédé d'ajustement de contraste ;
- la figure 34 montre des exemples de sous-images acquise à T1 et à T2 selon les conditions d'acquisition « backlight », « bottom » et « médian » après l'application d'un filtre médian ;
- la figure 35 montre un diagramme des étapes relatives à l'évaluation des premier et deuxième paramètres d'évolution selon la figure 31 ;
- la figure 36 montre un diagramme des étapes relatives à l'évaluation du paramètre de contraste selon un mode de réalisation de l'invention ;
- la figure 37 montre un diagramme des étapes relatives à l'évaluation du paramètre de corrélation selon un mode de réalisation de l'invention ;
- la figure 38 montre un diagramme des étapes relatives à la détermination de la croissance effective selon la figure 31 selon un mode de réalisation de l'invention ;
- la figure 39 montre une représentation graphique de la croissance de micro-organismes telle que détectée, en fonction des valeurs des paramètres de contraste et de corrélation.

## Description détaillée de l'invention

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après.

La présente invention concerne l'analyse d'un échantillon. Selon la présente invention, l'échantillon peut être de diverses origines, par exemple d'origine alimentaire, environnementale, vétérinaire, clinique, pharmaceutique ou cosmétique.

Parmi les échantillons d'origine alimentaire, l'on peut citer, de façon non-exhaustive, un échantillon de produits lactés (yaourts, fromages, etc.), de viande, de poisson, d'oeuf, de fruit, de légume, d'eau, de boisson (lait, jus de fruits, soda, etc.). Bien évidemment, ces échantillons d'origine alimentaire peuvent aussi provenir de sauces ou de plats plus élaborés ou des matières premières non transformées ou partiellement transformées. Un échantillon alimentaire peut également être issu d'une alimentation destinée aux animaux, telle que des tourteaux, des farines animales.

Tel qu'indiqué précédemment, l'échantillon peut être d'origine environnementale et peut consister, par exemple, en un prélèvement de surface, d'eau, d'air, etc.

L'échantillon peut également consister en un échantillon d'origine clinique, pouvant correspondre à des prélèvements de fluide biologique (urine, sang total, ou dérivés tel que sérum, salive, pus, liquide céphalo-rachidien, etc.), de selles (par exemple diarrhées cholériques), de prélèvements de nez, de gorge, de peau, de plaies, d'organes, de tissus ou de cellules isolées. Cette liste n'est évidemment pas exhaustive.

D'une manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon prélevé est, en général, susceptible de - ou suspecté de - contenir au moins un micro-organisme externe cible tel qu'une bactérie, une levure, un champignon.

Selon la présente invention, l'analyse est réalisée à l'aide d'un récipient d'analyse tel qu'une boîte de Petri comprenant un réceptacle et un couvercle. Le réceptacle et/ou le couvercle peuvent comprendre un identifiant. Le réceptacle comprend un milieu de culture tel qu'un milieu de culture gélosé. Un fluide est ensemencé sur le milieu de culture avant le début de l'analyse. Le but de l'analyse est de déterminer la présence de micro-organismes spécifiques et la concentration en micro-organismes au sein du fluide sur le milieu de culture, après incubation de la boîte de Petri.

Selon un mode de réalisation préféré de l'invention, le récipient d'analyse est une boîte de Petri qui comprend comme identifiant une étiquette latérale disposée sur la paroi latérale du réceptacle de la boîte de Petri.

Selon un mode de réalisation de l'invention, les conditions d'acquisition des images d'une boîte de Petri sont identiques. Dans ce mode de réalisation, l'éclairage et l'angle de vue de la boîte de Petri, lors de la capture d'images correspondantes à deux temps d'acquisition distincts, sont identiques pour chaque image acquise. Selon la présente invention, l'objectif de l'analyse d'images d'une boîte de Petri est de recaler une image acquise avant incubation de la boîte de Petri avec une image correspondante acquise après incubation de la boîte de Petri, ou deux images de la boîte de Petri acquises à des temps d'incubation différents. En effet, lors de la manipulation de la boîte de Petri, à l'aide d'un dispositif automatisé ou manuellement, la position de la boîte de Petri peut varier entre l'instant d'acquisition de l'image avant l'incubation et l'instant d'acquisition de l'image correspondante après incubation. Ainsi, la comparaison des deux images acquises à des instants différents permet de déterminer la présence ou l'absence de colonies de micro-organismes localisée au sein de la boîte de Petri. La boîte de Petri étant circulaire, le recalage peut nécessiter une rotation et/ou une translation de l'image de la boîte de Petri afin d'obtenir la correspondance entre les deux images.

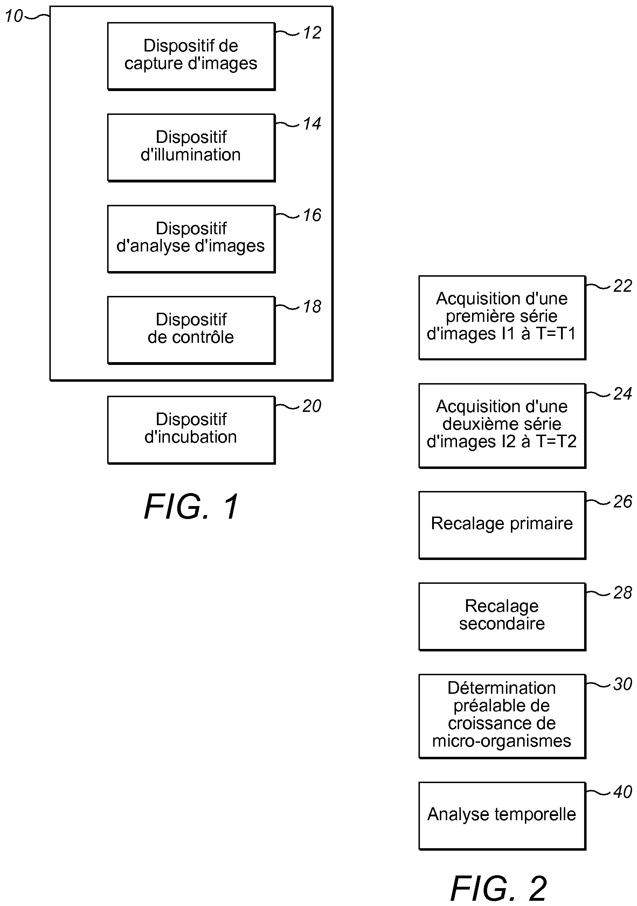

La figure 1 montre un système d'analyse d'images 10 comprenant un dispositif de capture d'image 12, un dispositif d'illumination 14, un dispositif d'analyse d'images 16 et un dispositif de contrôle 18.

Le dispositif de capture d'images 12 comprend un dispositif numérique tel qu'une caméra numérique afin d'acquérir des images de l'objet à analyser. Le dispositif de capture d'images 12 acquiert une première série d'images à un temps T1 et une deuxième série d'images à un temps T2. Le temps T1 correspond à un instant initial c'est-à-dire avant l'ensemencement de micro-organismes et avant translation, ou à un instant initial en présence de micro-organismes et après une première période d'incubation au sein de la boîte de Petri. Le temps T2 correspond à un instant ultérieur à l'instant initial T1, après incubation de la boîte de Petri, ou après une deuxième période d'incubation, c'est-à-dire, potentiellement, en présence de micro-organismes au sein de la boîte de Petri. A titre d'exemple, le dispositif de capture d'images 12 comprend un capteur d'images monochrome de type CCD consommant entre 12 et 24 Volts et utilisant la technologie de balayage avec une vitesse maximale d'acquisition de 17 images par seconde.

Le dispositif d'illumination 14 comprend par exemple une ou plusieurs sources lumineuses telles que des diodes électroluminescentes (DELs) afin d'illuminer l'objet à analyser selon des conditions déterminées par l'utilisateur. Les sources lumineuses sont disposées soit au-dessus, soit au-dessous de la boîte de Petri à analyser. Le dispositif d'illumination 14 permet de générer des conditions d'acquisition relatives à différentes possibilités d'illumination de la boîte de Petri telles qu'une illumination rouge, verte et/ou bleue (RVB) combinées à la présence ou à l'absence d'un cache situé au-dessous de la boîte de Petri, c'est-à-dire au-dessous du réceptacle ou au-dessous du couvercle de la boîte de Petri. Ainsi, l'illumination de type « backlight » correspond à l'acquisition d'images en présence d'une illumination arrière de la boîte de Petri, en l'absence d'un cache situé au-dessous de la boîte de Petri. L'illumination de type « bottom annular» ou «bottom» correspond à l'acquisition d'images en présence d'une illumination située au-dessus de la boîte de Petri, et selon quatre directions par rapport à la boîte de Petri, c'est-à-dire vers la gauche, vers la droite, vers la partie supérieure, et vers la partie inférieure de ladite boîte de Petri. L'illumination de type « left bottom » correspond à l'obtention d'images basée sur une illumination située au-dessus de la boîte de Petri de type « bottom » et du côté gauche de ladite boîte de Petri. L'illumination de type « median » correspond à l'obtention d'une image médiane basée sur les quatre images distinctes relatives à chaque direction, gauche, droite, partie inférieure, et partie supérieure de la boîte de Petri, obtenues au moyen d'une illumination « bottom». D'autres conditions d'acquisition peuvent être envisagées en utilisant une combinaison de sources lumineuses disposées selon une direction spécifique par rapport à la boîte de Petri en présence ou en absence d'un cache situé au-dessous de la boîte de Petri afin d'obtenir un objet visible et sans artefact en fonction de la nature de l'échantillon et des micro-organismes à identifier.

Le dispositif d'illumination 14 peut également produire des illuminations verticales ou annulaires par rapport à la boîte de Petri à analyser.

Le dispositif d'analyse d'images 16 comprend notamment un support d'objet et des moyens de maintien de l'objet tel qu'une pince afin de maintenir un objet tel qu'une boîte de Petri.

Le dispositif de contrôle 18 comprend notamment un micro-processeur (non montré), un écran d'affichage (non montré), une mémoire de stockage (non montrée), afin d'exécuter des algorithmes de traitement d'images. Le dispositif de contrôle 18 permet de définir les paramètres de fonctionnement du dispositif de capture d'images 12, du dispositif d'illumination 14 et du dispositif d'analyse d'images 16 afin de contrôler le fonctionnement du dispositif de capture d'images 12, du dispositif d'illumination 14 et du dispositif d'analyse d'images 16.

Le système d'analyse d'images 10 fonctionne en association avec un dispositif d'incubation 20. Le dispositif d'incubation 20 permet d'incuber au moins une boîte de Petri selon des conditions d'incubation spécifiques concernant la durée et la température, afin de favoriser la croissance de micro-organismes au sein des boîtes de Petri.

Ainsi, selon un fonctionnement classique, et en considérant une seule boîte de Petri à titre d'exemple, la boîte de Petri ensemencée avec un échantillon est acheminée via un moyen de transport connu selon l'état de l'art, tel qu'un tapis roulant, au sein du dispositif d'incubation 20.

A l'issue de la période d'incubation, la boîte de Petri est à nouveau acheminée au sein du système d'analyse d'images 10.

Après le traitement des images obtenues au moyen du système d'analyse d'images 10, un système d'identification (non montré) permet de déterminer, selon un procédé manuel ou automatisé appliqué aux images acquises, la nature et le nombre de colonies de micro-organismes qui se sont éventuellement développées au sein de la boîte de Petri.

La présente invention concerne l'analyse d'images disponibles au sein du système d'analyse d'images 10, après incubation d'une boîte de Petri et acquisition des images de ladite boîte de Petri.

Au sein de la présente description, à titre d'exemple, l'objet considéré est une boîte de Petri. La présente invention s'applique à une pluralité d'images ou séries d'images concernant une ou plusieurs boîtes de Petri. Une première série d'images I1 est acquise à un temps T=T1 et une deuxième série d'images I2 est acquise à un temps T=T2. La présente description, à titre d'exemple, fait référence à une image I1 issue de la première série d'images I1 acquise à T1 et à une image I2 issue de la deuxième série d'images I2 acquise à T2.

Comme montré sur la figure 2, le procédé global selon la présente invention comprend une première étape d'acquisition 22 de la première série d'images I1, à un temps T1, une deuxième étape d'acquisition 24 de la deuxième série d'images I2, à un temps T2, ainsi qu'une étape 26 relative à un premier procédé de recalage primaire, une étape 28 relative à un deuxième procédé de recalage secondaire, une étape 30 relative à un troisième procédé de détection préalable de micro-organismes. Dans l'étape 22, les images sont acquises selon un procédé décrit au sein de la demande internationale publiée sous le numéro WO2012/152769. Les premier, deuxième et troisième procédés sont distincts et leurs algorithmes respectifs sont stockés au sein de la mémoire de stockage du dispositif de contrôle 18.

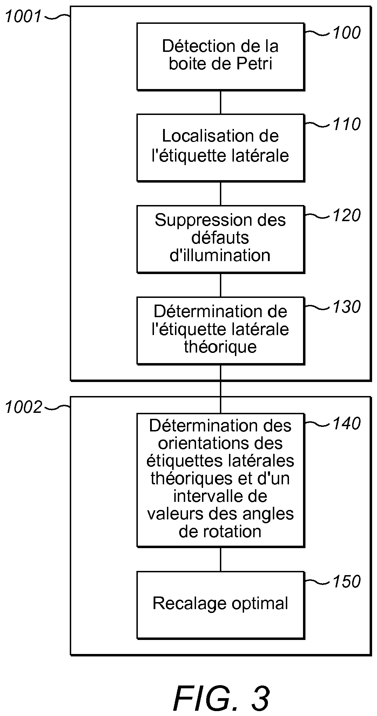

Le premier procédé concerne le recalage primaire ou recalage grossier montré sur la figure 3 et relatif au positionnement de la boîte de Petri entre la première image I1 acquise à T1 et la deuxième image I2 acquise à T2.

Le procédé de recalage primaire est basé sur la détection de l'étiquette latérale de la boîte de Petri. Ainsi, le procédé de recalage primaire comprend, comme montré sur la figure 3, un procédé de détection de l'étiquette latérale 1001 et un procédé de détermination des paramètres de recalage primaire 1002 associés aux caractéristiques de l'étiquette latérale.

Le procédé de détection de l'étiquette latérale 1001 comprend l'utilisation d'au moins deux images de la boîte de Petri, avec au moins une image acquise en utilisant la condition d'illumination « left bottom » et au moins une image acquise en utilisant la condition d'illumination « backlight ».

Le procédé de détection de l'étiquette latérale 1001 comprend une première étape 100 concernant la détection de la boîte de Petri. La première étape 100 comprend une détection des bords de la boîte de Petri, selon une méthode connue dans l'état de l'art.

Les images utilisées dans la première étape correspondent aux images acquises au temps T1 et au temps T2 avec la condition d'illumination « backlight ». Ainsi, la première étape permet de déterminer les coordonnées du centre C et la longueur du rayon R de la boîte de Petri, et ce pour chaque image acquise.

Dans les étapes suivantes, seuls les pixels situés à l'intérieur d'un disque caractérisé par les valeurs ainsi déterminées de R et C sont considérés au sein des images acquises.

Le procédé de détection de l'étiquette latérale comprend une deuxième étape 110 concernant la localisation de l'étiquette latérale.

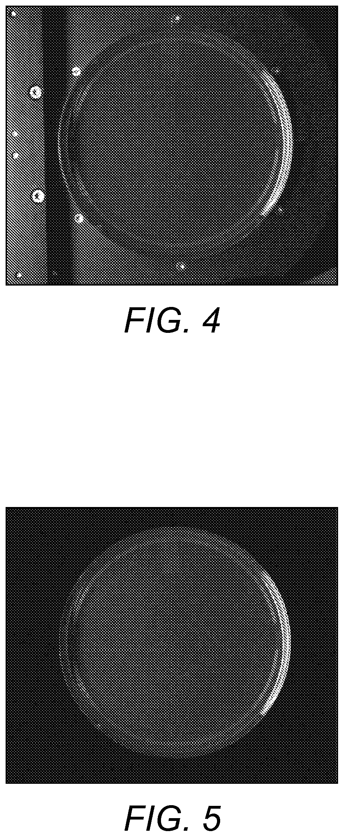

Les images utilisées dans la deuxième étape 110 correspondent aux images acquises au temps T1 comme montré sur la figure 4, et au temps T2 avec la condition d'illumination « left bottom». En effet l'étiquette latérale localisée sur le côté droit de la boîte de Petri est visible, de manière optimale, selon cette condition d'illumination.

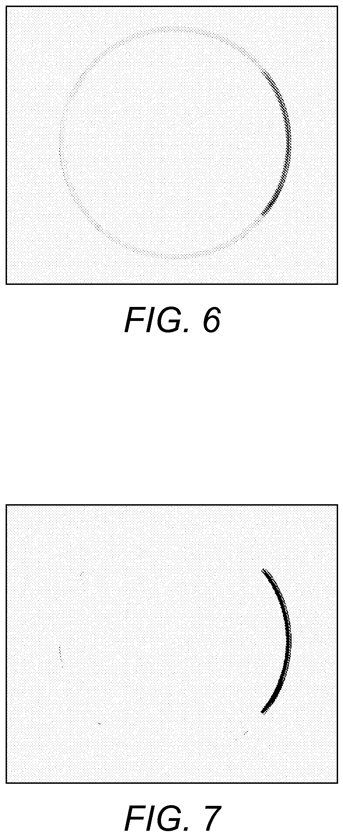

La deuxième étape 110 comprend la création d'un premier disque de rayon R1 = R-d1 et la création d'un deuxième disque de rayon R2=R-d2, d1 et d2 étant des valeurs déterminées au préalable par l'utilisateur, avec d1<d2, telles que d1=10 pixels et d2=50 pixels. Pour une boîte de Petri de rayon R=950 pixels, le premier disque, comme montré sur la figure 5, correspond à un rayon R1=940 pixels et le deuxième disque, correspond à un rayon R2=900 pixels. La deuxième étape comprend la création d'un anneau résultant contenant les pixels situés à une distance d comprise entre R1 et R2, soit entre 900 et 940 pixels, comme montré sur la figure 6.

Dans les étapes suivantes, seuls les pixels situés à l'intérieur de l'anneau résultant sont considérés au sein des images acquises.

La deuxième étape 110 comprend ensuite l'application d'une opération de segmentation ou binarisation sur l'image acquise de la boîte de Petri. En effet, dans la mesure où l'étiquette latérale se distingue par une portion claire d'anneau du reste de l'anneau résultant, l'opération de segmentation permet d'isoler efficacement l'étiquette latérale. L'opération de segmentation peut être utilisée en appliquant un algorithme tel que l'algorithme des k-moyennes ou « k-means » du logiciel Matlab™ basé sur une recherche de deux groupes ou « deux clusters » en considérant, pour un cluster, un centroïde initialisé à la valeur minimale du triplet RVB pour approcher la valeur du blanc et regrouper ainsi les pixels clairs et, pour un autre cluster, un centroïde initialisé à la valeur maximale du triplet RVB pour approcher la valeur du noir, et regrouper ainsi les pixels foncés.

L'opération de segmentation permet de repérer différents objets au sein des images acquises, comme montré sur la figure 7. Ces objets comprennent par exemple l'étiquette latérale et les défauts de réflexion.

Les défauts de réflexion sont générés par la réflexion des faisceaux lumineux du dispositif d'illumination 14 sur les bords de la boîte de Petri. Les défauts de lumière ou « barbules » ont une forme généralement étroite et incurvée.

L'étiquette latérale comprend éventuellement des défauts d'illumination relatifs à une surexposition lors de l'illumination de la boîte de Petri.

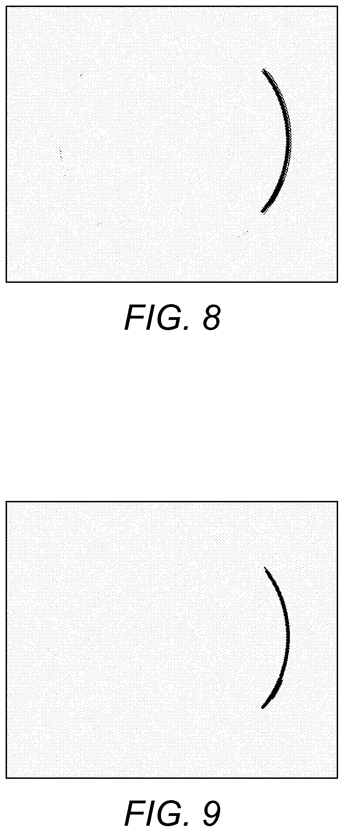

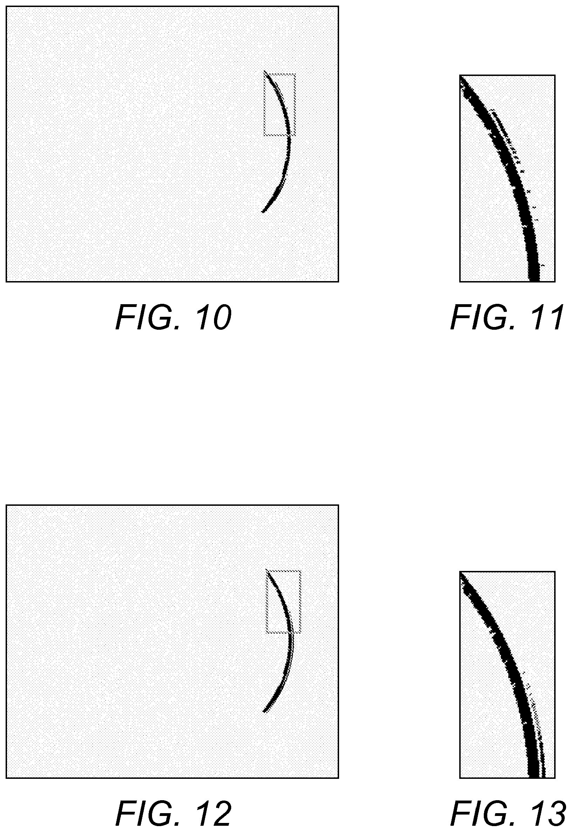

De manière générale, l'étiquette latérale est formée d'une seule pièce. Cependant, l'étiquette latérale peut être parfois constituée de différents fragments, de manière volontaire ou de manière involontaire. Ainsi, selon le type de conditionnement des boîtes de Petri ou en fonction d'incidents liés par exemple à la qualité du transport des boîtes de Petri, un fragment de l'étiquette latérale peut s'avérer manquant. Une étape de suppression des défauts d'illumination ne permet pas toujours de distinguer l'étiquette latérale des défauts d'illumination lorsque la taille de l'étiquette latérale est du même ordre de grandeur que la taille des défauts d'illumination. Ainsi, le procédé de détection de l'étiquette latérale comprend une troisième étape 120 concernant la suppression des défauts d'illumination de l'étiquette latérale. La troisième étape 120 comprend l'application d'une fonction d'ouverture morphologique, selon l'art antérieur, sur l'image binarisée, montrées sur les figures 8 et 9. La fonction d'ouverture correspond à la combinaison d'une fonction d'érosion et d'une fonction de dilatation. Pour l'application de la fonction d'ouverture, la fonction STREL du logiciel Matlab™ est utilisée afin de définir un élément structurant spécifique comprenant un segment de longueur déterminée, par exemple 10 pixels, concernant un nombre défini de pixels voisins du pixel considéré, par exemple 3 pixels, associé à un angle de rotation dudit élément structurant compris entre -90 et 90 degrés. Comme montré sur les figures 10 et 11, la valeur de l'angle de rotation est 0°. Comme montré sur les figures 12 et 13, la valeur de l'angle de rotation est 45°.

La fonction d'érosion, avec les valeurs définies ci-dessus, permet d'éliminer les objets ayant une épaisseur inférieure à 10 pixels quelle que soit la courbure de ces objets.

La fonction de dilatation permet d'obtenir une correction de la courbure de l'étiquette latérale, afin d'obtenir une courbure globale de forme convexe.

La fonction d'ouverture est appliquée pour chaque canal de couleur du triplet RVB.

Ainsi, à l'issue de l'application de la fonction d'ouverture, les défauts d'illumination de l'étiquette latérale sont supprimés sur les images acquises au temps T1 et au temps T2.

Ensuite, le procédé de détection de l'étiquette latérale comprend la quatrième étape 130 relative à la détermination d'une étiquette latérale théorique formée d'un seul fragment. La zone de la boîte de Petri associée à l'étiquette latérale théorique correspond à un arc de cercle de sommet situé au centre de la boîte de Petri, de largeur 20 pixels et avec un angle d'étalement θ.

L'angle d'étalement θ de l'étiquette latérale théorique est déterminé par la valeur maximale θmax et la valeur minimale θmin de θ afin de définir la surface d'étalement recouvrant l'ensemble des fragments de l'étiquette latérale détectés à l'étape précédente.

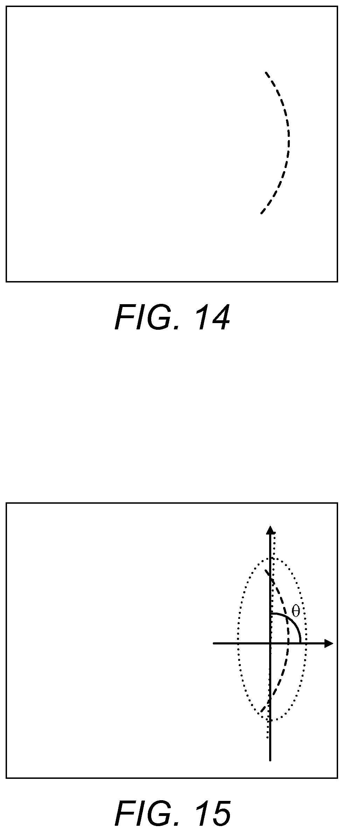

La quatrième étape 130 permet d'obtenir une étiquette latérale théorique, comme montré sur la figure 14. L'étiquette latérale théorique est située sur le pourtour de la boîte de Petri, comme montré sur la figure 5, et contient tous les fragments de l'étiquette latérale.

Ainsi, le procédé de détermination des paramètres de recalage primaire 1002 peut être appliqué comme décrit ci-dessous.

Le procédé de détermination des paramètres de recalage primaire 1002 comprend une première étape 140 concernant la détermination des orientations des étiquettes latérales et la détermination d'un intervalle de valeurs des angles de rotation.

Pour définir une plage de recherche pour l'angle de rotation, on utilise l'orientation des étiquettes latérales théoriques au temps T1 et au temps T2.

De manière connue, la fonction « regionprops » associée à la propriété « Orientation » du logiciel Matlab™ permet de déterminer l'orientation d'objets au sein d'une image binarisée. L'orientation de l'étiquette théorique latérale est déterminée en obtenant les angles θ1 et θ2. Cette orientation correspond à l'orientation de l'ellipse ayant le même moment d'inertie que celui de l'étiquette latérale théorique, comme montré sur la figure 15.

Pour définir la plage de recherche pour l'angle de rotation, l'étiquette latérale théorique est utilisée selon trois aspects différents.

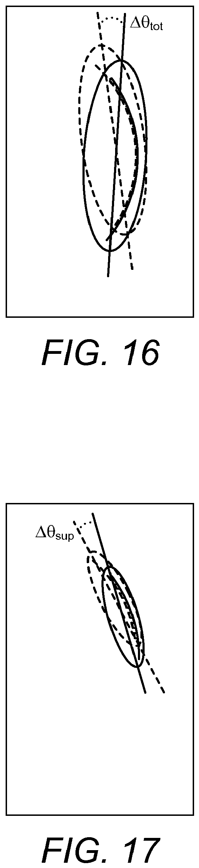

En effet, selon un premier aspect, montré sur la figure 16, le calcul de l'orientation de l'étiquette latérale théorique est basé sur la totalité de l'étiquette latérale théorique afin d'obtenir les angles correspondants θ1tot et θ2tot et l'écart angulaire Δθtot.

Selon un deuxième aspect, montré sur la figure 17, le calcul de l'orientation de l'étiquette latérale théorique est basé sur la moitié supérieure de l'étiquette latérale théorique afin d'obtenir les angles correspondants θ1sup and θ2sup et l'écart angulaire correspondant Δθsup.

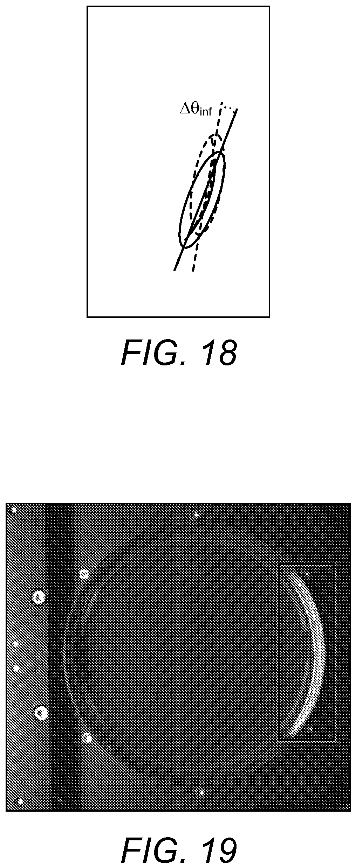

Selon un troisième aspect, montré sur la figure 18, le calcul de l'orientation de l'étiquette latérale théorique est basé sur la moitié inférieure de l'étiquette latérale théorique afin d'obtenir les angles correspondants θ1inf and θ2inf et l'écart angulaire correspondant Δθinf.

Pour chacun des trois aspects, les écarts entre les angles d'orientation à T1 et à T2 sont calculés comme indiqués ci-dessous :

$$\Delta\theta tot = |\theta 1 tot - \theta 2 tot|,$$

$$\Delta\theta sup = |\theta 1 sup - \theta 2 sup|,$$

$$\Delta\theta inf = |\theta 1 inf - \theta 2 inf|,$$

Un intervalle des valeurs possibles de l'angle d'orientation optimal θopt est défini ci-dessous :

Intervalle optimal : [min (Δθtot, Δθsup, Δθinf) – 0,2°; max (Δθtot, Δθsup, Δθinf) + 0,2°].

Le procédé de détermination des paramètres de recalage primaire 1002 comprend une deuxième étape 150 de recalage optimal concernant la rotation et la translation de la boîte de Petri entre les temps T1 et T2, le centre de la rotation étant assimilé au centre de la boîte de Petri.

L'étape de recalage optimal permet de déterminer, à partir de l'image acquise au temps T2, des paramètres optimaux de recalage primaire comprenant un angle de rotation et un vecteur de translation afin d'appliquer ces paramètres à l'image acquise au temps T1 pour que les deux images acquises soient superposables.

La détermination des paramètres optimaux est obtenue en utilisant une fonction de corrélation croisée 2D, telle que disponible dans le logiciel Matlab™.

Pour estimer les paramètres optimaux, on test toutes les valeurs d'angle de rotation dans l'intervalle optimal avec un pas de 0,1° de l'image à T2 et on calcul la corrélation croisée entre l'image à T2 tournée et l'image à T1. Chaque valeur résultante représente la translation optimale pour chaque valeur d'angle de rotation dans l'intervalle optimal. Le maximum de ces valeurs résultantes permet de déterminer la rotation optimale et sa translation optimale associée.

Le deuxième procédé du procédé global de l'invention concerne le recalage secondaire ou recalage fin 28. Le deuxième procédé peut éventuellement être réalisé en premier lieu, sans que le premier procédé relatif au recalage primaire n'ait été réalisé au préalable.

La figure 19 montre une première image ou image initiale d'une boîte de Petri, acquise au moyen du système d'analyse d'images 10 à un temps T1, avant l'incubation de la boîte de Petri par exemple.

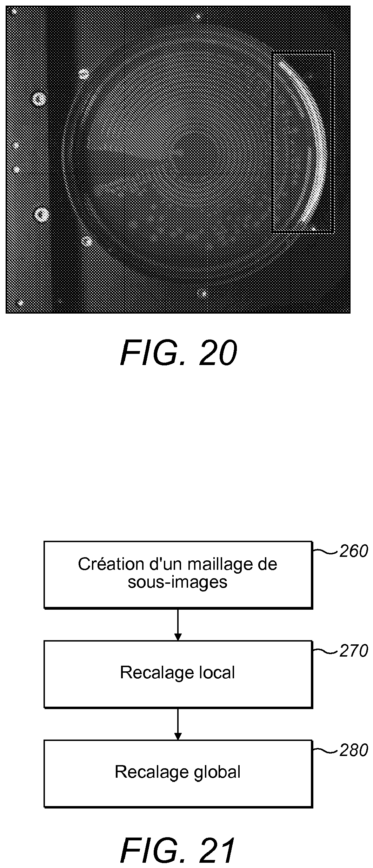

La figure 20 montre une deuxième image ou image finale de la boîte de Petri montrée sur la figure 19 et acquise à un temps T2 après l'incubation de la boîte de Petri au sein du dispositif d'incubation 20. Comme montré sur la figure 20, des colonies de micro-organismes sont présentes au sein de la boîte de Petri au temps T2.

Comme montré sur la figure 21, le procédé de recalage secondaire comprend une étape 260 relative à la création d'un maillage de sous-images au sein de l'image initiale I1 acquise au temps T1 et de l'image finale I2 acquise au temps T2, une étape 270 relative à un procédé de recalage local et une étape 280 relative à un procédé de recalage global.

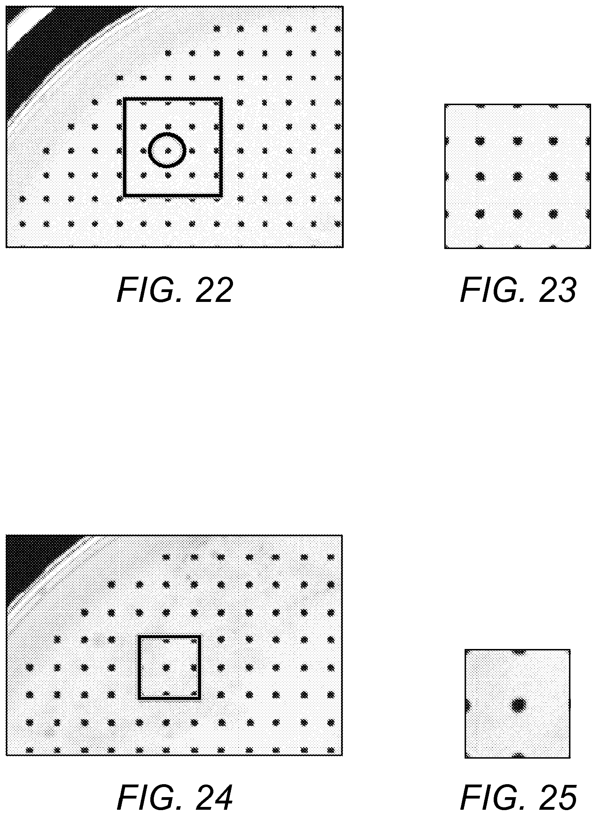

Pour l'étape 260 de création d'un maillage de sous-images, une image initiale I1 est quadrillée, comme montré sur la figure 22, selon un maillage initial qui comprend des cellules initiales. La figure 23 montre un exemple d'une cellule initiale. L'image finale I2 est quadrillée comme montré sur la figure 24 selon un maillage final qui comprend des cellules finales. La figure 25 montre un exemple d'une cellule finale. La dimension d'une cellule initiale est supérieure à la dimension d'une cellule finale.

Comme montré sur la figure 21, le procédé de recalage secondaire comprend une étape 270 de recalage local concernant chaque couple de sous-images obtenu à l'issue de l'étape 260 décrite ci-dessus.

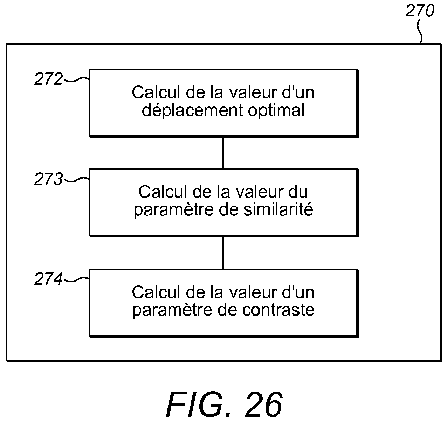

L'étape 270 de recalage local, montré sur la figure 26, comprend une sous-étape 272 pour calculer la valeur d'un déplacement optimal entre les deux sous-images considérées au sein du couple de sous-images. Le déplacement optimal est estimé en considérant le déplacement de la cellule finale sur la cellule initiale correspondante. Dans la mesure où la dimension de la cellule finale est inférieure à la dimension de la cellule initiale, le déplacement de la cellule finale est possible selon une marge de déplacement paramétrée par l'utilisateur. Pour chaque déplacement une valeur d'un paramètre de similarité est calculée dans une étape 273. Ce paramètre de similarité peut être, par exemple, la mesure de distance ou d'information mutuelle. Dans la présente invention, le paramètre de similarité correspond au coefficient de corrélation. Pour chaque déplacement de la sous-image finale sur la sous-image initiale, une valeur du coefficient de corrélation est obtenue. La valeur maximale de ce coefficient de corrélation définit le déplacement optimal pour un couple de sous-images.

La sous-étape 274 permet de calculer la valeur d'un paramètre de contraste entre les deux sous-images considérées au sein du couple de sous-images recalées selon le déplacement optimal estimé dans la sous-étape 272. Le second paramètre de contraste permet d'évaluer la quantité d'information présente au sein de chaque sous-image du couple de sous-images. Dans la présente invention, la valeur du paramètre de contraste est calculée comme l'écart-type de la distance entre les couleurs calculée pixel à pixel selon la formule ci-dessous :

$$\sigma_X = \sqrt{E\left[(X - E[X])^2\right]} = \sqrt{E[X^2] - E[X]^2}$$

Le paramètre de contraste permet de déterminer les sous-images qui sont pertinentes pour le calcul ultérieur dans le procédé de recalage global.

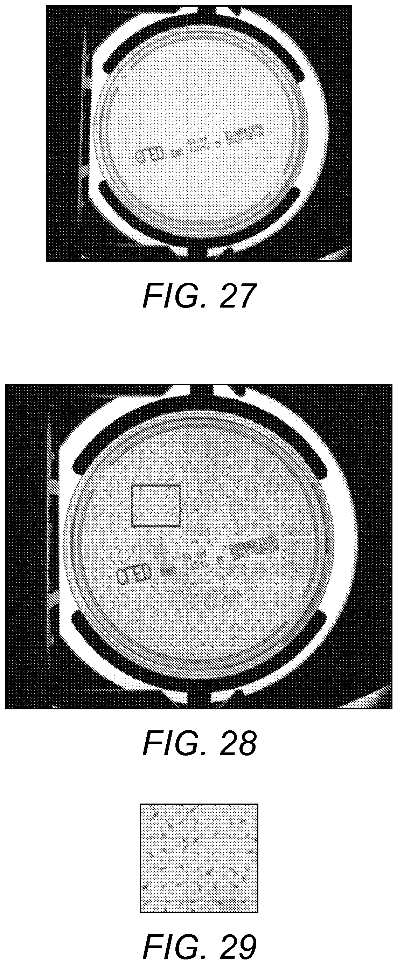

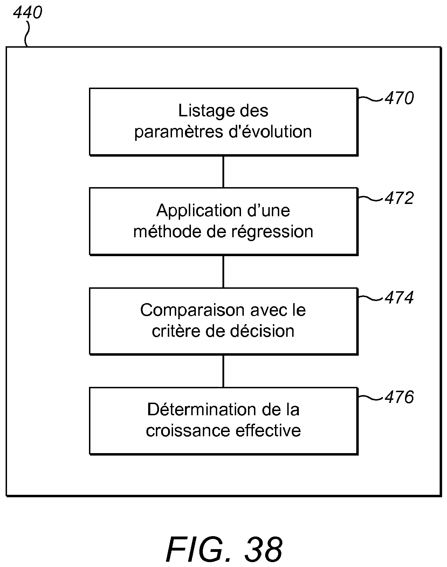

Ainsi l'étape 270 permet d'obtenir un ensemble de transformations locales en translation entre l'image initiale acquise à T1 montrée sur la figure 27 et l'image finale acquise à T2 montrée sur la figure 28 par des vecteurs pour chaque point du maillage. La figure 29 montre en détail le contenu du carré représenté sur la figure 38. Comme montré sur les figures 28 et 29, ces transformations locales sont représentées par des vecteurs.

Comme montré sur la figure 21, le procédé de recalage d'images selon l'invention comprend une étape 280 de recalage global. L'étape 280 permet d'utiliser les résultats de l'étape 270 afin d'en déduire, pour chaque couple d'images I1 et I2, des paramètres de recalage global.

La détermination d'une transformation globale est réalisée sur la base, d'une part, de l'ensemble des transformations locales c'est-à-dire des translations et, d'autre part, des paramètres de similarité et de contraste pour tous les couples de sous-images.

Les valeurs des paramètres de similarité et de contraste permettent de sélectionner les couples de sous-images les plus pertinents. Ainsi, les transformations locales les plus pertinentes correspondantes peuvent être déduites, par exemple, en appliquant une étape de comparaison avec une valeur seuil.

Ensuite, une transformation globale est déterminée à partir des transformations locales les plus pertinentes par exemple en considérant la moyenne, la médiane ou la transformation locale la plus fréquemment représentée parmi les transformations locales.

Comme montré sur la figure 2, le procédé global selon l'invention comprend une étape 30 de détermination préalable de croissance de micro-organismes sur l'image I2 acquise au temps T=T2.

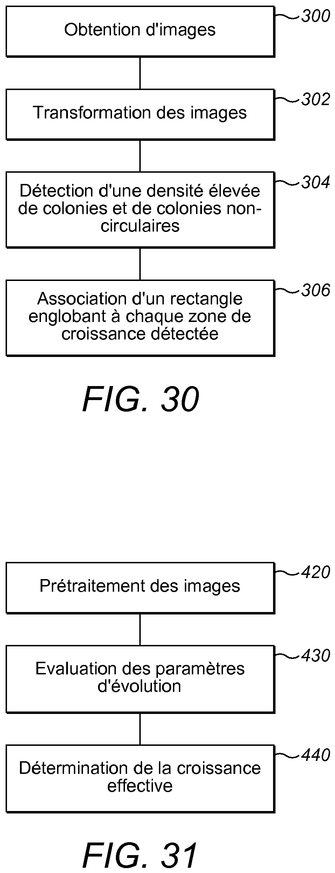

L'étape 30 de la figure 2 comprend l'application d'un procédé de détermination préalable de croissance afin de détecter l'éventuelle croissance de micro-organismes au sein de la boîte de Petri après incubation de ladite boîte de Petri. Le procédé de détermination préalable de croissance comprend un procédé d'identification de zones potentielles de croissance et un procédé d'analyse temporelle desdites zones de croissance. Comme montré sur la figure 30, le procédé de détermination préalable de croissance comprend une étape 300 relative à l'obtention d'une ou plusieurs images de la boîte de Petri, à l'issu du procédé de recalage secondaire, selon différentes conditions d'acquisition relatives notamment au dispositif d'illumination 14.

Le procédé de détermination préalable de croissance comprend également une étape 302 pour créer une image numérisée à partir des images obtenues lors de l'étape 300.

Le procédé de détermination préalable de croissance comprend une étape 304 pour détecter la présence d'une densité élevée de colonies de micro-organismes ainsi que les colonies de micro-organismes de forme non-circulaire. Ainsi, l'étape 304 permet de détecter une zone de croissance de micro-organismes, la forme de ladite zone de croissance étant variable. Afin de faciliter l'application ultérieure des calculs, ladite zone de croissance est définie au moyen d'un rectangle dit « rectangle englobant ». Le « rectangle englobant » est centré sur la zone de croissance et permet d'agrandir la surface de la zone de croissance selon une dimension déterminée à l'avance, par exemple 10 pixels autour de la zone de croissance.

Le procédé de détermination préalable de croissance comprend ensuite une étape 306 pour associer un élément de localisation tel qu'un rectangle englobant, à chaque élément de croissance déterminé par le procédé de détermination préalable de croissance.

Ainsi, le procédé de détermination préalable de croissance de micro-organismes permet de déterminer si des colonies de micro-organismes sont présentes au sein de la boîte de Petri.

Afin d'améliorer les résultats issus du procédé de détermination préalable, un procédé d'analyse temporelle 40 de la croissance de micro-organismes est ensuite appliqué afin de valider chaque rectangle englobant une zone de croissance obtenue lors de l'application du procédé de détermination préalable de croissance de micro-organismes et obtenir ainsi l'indication de la croissance effective de micro-organismes au sein de la boîte de Petri.

Le procédé d'analyse temporelle considère donc les images obtenues après l'application du procédé de recalage primaire optionnel et du procédé de recalage secondaire et, pour lesquelles, l'application du procédé de détermination préalable de croissance a généré des rectangles englobant des zones potentielles de croissance au sein des images correspondantes.

Selon le mode de réalisation de la présente invention, les images initiales et les images finales considérées correspondent à des images acquises avec les trois conditions d'acquisition suivantes : « backlight », « bottom» et « median ».

En effet, chaque condition d'acquisition du dispositif d'illumination 14 permet une observation spécifique de la boîte de Petri. Ainsi, l'illumination de type « backlight » permet d'observer par transparence les éléments contenus au sein de la boîte de Petri.

Les conditions d'acquisition de type «bottom» et «médian» permettent d'observer les éléments localisés à la surface du milieu de culture gélosé au sein de la boîte de Petri.

Comme montré sur la figure 31, le procédé d'analyse temporelle comprend une étape 420 pour appliquer un procédé de prétraitement d'images aux images initiales et aux images finales, une étape 430 pour évaluer des critères d'évolution basés sur les images initiales et les images finales et une étape 440 pour déterminer la croissance effective de micro-organismes.

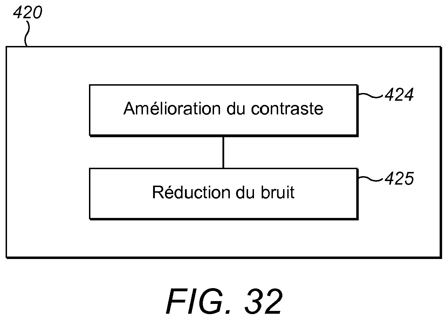

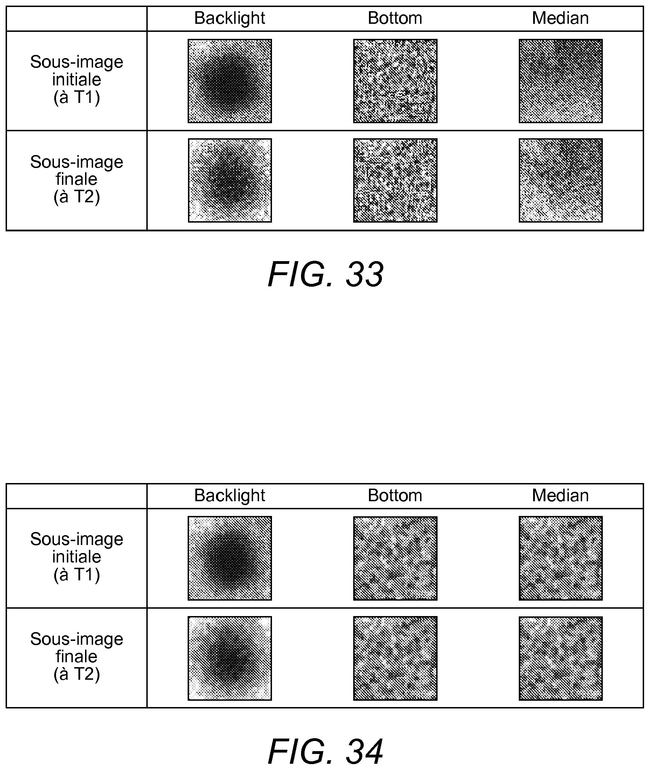

L'étape 420, relative au procédé de prétraitement d'images et montré sur la figure 32, comprend une sous-étape 424 pour améliorer le contraste au sein de chaque zone relative à rectangle englobant de ladite zone au sein des sous-images. Ainsi, la sous-étape 424 concerne l'application d'un procédé d'ajustement de contraste d'une image tel que l'égalisation d'histogramme connue dans l'art antérieur. Ainsi, les images obtenues après l'étape 424 contiennent différents niveaux de gris comme montré sur la figure 33, en fonction des différentes conditions d'illumination, c'est-à-dire « backlight », « bottom » et « median ».

L'étape 420 comprend ensuite une sous-étape 425 pour réduire, au sein de l'image, le bruit généré par l'augmentation de contraste. Ainsi, la sous-étape 425 concerne l'application d'un filtre médian tel que connu dans l'art antérieur, par exemple un filtre pour une fenêtre 3x3.

Ainsi, à l'issue de la sous-étape 425, les zones relatives aux rectangles englobant les zones potentielles de croissance contiennent des transitions montrées sur la figure 34, qui sont plus lisses et plus homogènes que les transitions desdites zones montrées sur la figure 33, à l'issue de l'étape 424.

Comme montré sur la figure 31, le procédé d'analyse temporelle comprend une étape 430 pour déterminer, au sein des sous-images, la valeur d'au moins un paramètre d'évolution.

Selon un mode de réalisation préféré de l'invention, la valeur de deux paramètres d'évolution doit être déterminée pour chaque ensemble de sous-images.

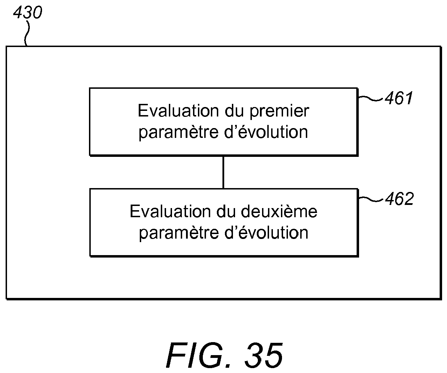

L'étape 430 comprend une sous-étape 461 montrée sur la figure 35 et relative au premier paramètre d'évolution qui permet de caractériser l'évolution de la couleur au sein des sous-images entre les temps d'acquisition T1 et T2. Selon un mode de réalisation de l'invention, le premier paramètre d'évolution correspond à un paramètre de contraste qui permet d'évaluer la stabilité globale de la sous-image entre les temps d'acquisition T1 et T2. Selon un mode de réalisation de l'invention, le premier paramètre d'évolution correspond à un paramètre de corrélation. L'étape 430 comprend une sous-étape 462 montrée sur la figure 35 et relative au deuxième paramètre d'évolution

Selon un mode de réalisation de l'invention, la méthode de mesure de contraste est basée sur le calcul de la déviation standard, par exemple au moyen de la fonction « std » du logiciel Matlab™, concernant les distances mesurées, par exemple au moyen de la fonction « pdist » du logiciel Matlab™, entre deux pixels, et ce pour tous les pixels localisés à l'intérieur du rectangle englobant :

$$C = std(pdist(array))$$

Pour la mesure du contraste entre des voxels dans une sous-image en couleur, la variable "array" correspond à un vecteur colonne n x 3.

Pour la mesure du contraste entre des pixels dans une sous-image en niveaux de gris, la variable "array" correspond à un vecteur colonne n.

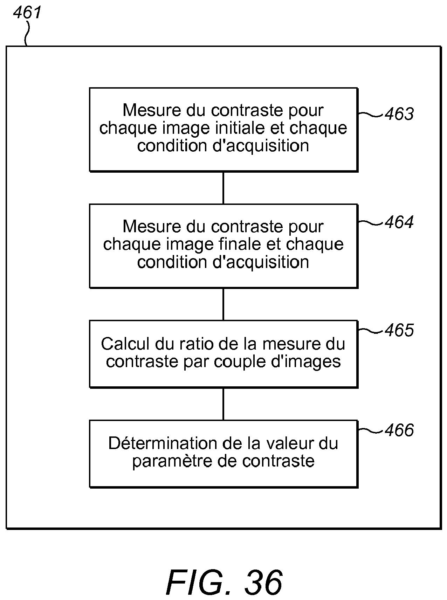

Comme montré sur la figure 36, la sous-étape 461 relative au calcul du paramètre de contraste comprend une pluralité d'étapes basées sur la mesure du contraste définie avec l'équation C ci-dessus, selon un mode de réalisation de l'invention.

La première étape 463 correspond à la mesure du contraste pour chaque sous-image d'une image initiale et pour chaque condition d'illumination « backlight », « bottom» et « median ».

La deuxième étape 464 correspond à la mesure du contraste pour chaque sous-image d'une image finale et pour chaque condition d'illumination « backlight », « bottom» et « median ».

La troisième étape 465 correspond au calcul des ratios des mesures de contraste en considérant deux à deux les mesures de contraste d'une sous-image aux temps T1 et T2, pour chaque condition d'illumination.

La quatrième étape 466 correspond à la détermination de la valeur minimale des ratios issus de l'étape 465 pour déterminer la valeur du paramètre de contraste.

La sous-étape 461 correspond à l'application de l'équation ContC ci-dessous :

$$ContC = \min\left(\frac{C_{\{T_1\ backlight\}}}{C_{\{T_2\ backlight\}}}, \frac{C_{\{T_1\ bottom\}}}{C_{\{T_2\ bottom\}}}, \frac{C_{\{T_1\ median\}}}{C_{\{T_2\ median\}}}\right)$$

Selon un autre mode de réalisation de l'invention, une autre équation ContC_alt peut être appliquée en utilisant les mêmes mesures de contraste :

*ContC_alt*

$$= \min(\frac{\min(C_{\{T_1 backlight\}}, C_{\{T_1 backlight\}})}{\max(C_{\{T_2 backlight\}}, C_{\{T_2 backlight\}})}, \frac{\min(C_{\{T_1 bottom\}}, C_{\{T_1 bottom\}})}{\max(C_{\{T_2 bottom\}}, C_{\{T_2 bottom\}})}, \frac{\min(C_{\{T_1 median\}}, C_{\{T_1 median\}})}{\min(C_{\{T_2 median\}}, C_{\{T_2 median\}})})$$

L'équation ContC_alt présente des avantages par rapport à l'équation ContC. En effet, les valeurs obtenues avec l'équation ContC_alt sont toujours comprises dans l'intervalle [0 ; 1]. En effet, pour un élément stable, c'est-à-dire un élément qui n'a pas évolué au cours du temps, la valeur obtenue avec l'équation ContC_alt est proche de 1, selon une marge d'appréciation que l'utilisateur peut fixer. Ainsi, pour une marge d'appréciation de 0,2, la valeur obtenue pour un élément stable est comprise dans l'intervalle [0,8 ; 1]. Pour un élément de croissance, c'est-à-dire un élément ayant évolué au cours du temps, la valeur obtenue avec l'équation ContC_alt est proche de 0, selon une marge d'appréciation que l'utilisateur peut fixer. Ainsi, pour une marge d'appréciation de 0,2, la valeur obtenue pour un élément ayant évolué est comprise dans l'intervalle [0 ; 0,2].

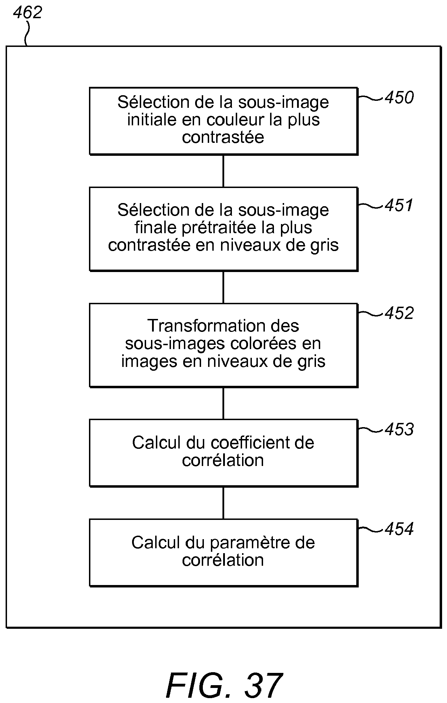

Comme montré sur la figure 37, la sous-étape 462 comprend une pluralité d'étapes. Ainsi, la première étape 450 correspond à la sélection de la sous-image initiale en couleur la plus contrastée, parmi les sous-images identiques acquises selon les trois conditions d'acquisition « backlight », « bottom» et « median ».

La deuxième étape 451 correspond à la sélection d'une sous-image finale prétraitée correspondante et telle qu'obtenue à l'issue des étapes 424 et 425.

La sélection de l'image la plus contrastée au sein des étapes 450 et 451 est basée sur la méthode de mesure de contraste décrite au sein de l'étape 461.

La troisième étape 452 comprend la transformation de la sous-image colorée en sous-image en niveaux de gris.

La quatrième étape 453 comprend le calcul du coefficient de corrélation entre la sous-image acquise à T1 et la sous-image acquise à T2, toutes deux en niveaux de gris, en utilisant la fonction « corr2 » du logiciel Matlab™.

La cinquième étape 454 correspond au calcul de la valeur du paramètre de corrélation qui correspond à la valeur maximale entre les deux valeurs de coefficients de corrélation calculées à l'étape 453, au moyen de l'équation ci-dessous :

$$CorrP = \max[corr2(T1gris, T2gris), corr2 (T1améliorée, T2améliorée)]$$

Comme montré sur la figure 31, le procédé d'analyse temporelle comprend une étape 440 pour déterminer la croissance effective de micro-organismes au sein de la boîte de Petri, à l'issue d'une période déterminée d'incubation.

L'objectif de l'étape 440 consiste à vérifier si l'élément repéré par une zone de croissance déterminée lors de l'application du procédé de détermination préalable de croissance au sein d'une sous-image, lors de l'étape 30, correspond effectivement à un élément de croissance tel qu'une colonie ou un ensemble de colonies de micro-organismes.

Comme montré sur la figure 38, l'étape 440 comprend une étape 470 afin de lister les différentes valeurs obtenues pour les deux paramètres d'évolution lors de l'étape 430. Ces valeurs sont associées à chaque rectangle englobant des zones potentielles de croissance de la boîte de Petri pour le temps d'acquisition T2

L'étape 440 comprend ensuite une étape 472 relative à l'application d'une méthode de régression logistique. Cette méthode permet d'obtenir une séparation entre deux groupes de données à discriminer sur la base de plusieurs paramètres au moyen d'une droite de régression définie. Au sein de la présente invention, la méthode de régression logistique utilisée permet de distinguer deux groupes de données. Le premier groupe de données concerne les zones potentielles de croissance qui sont des zones de croissance avérées, c'est-à-dire des zones positives. Le deuxième groupe de données concerne les zones potentielles de croissance qui ne correspondent pas à des zones de croissance. Il s'agit de zones négatives. La méthode de régression logistique au sein de la présente invention est basée sur deux paramètres, CorrP et ContC, selon l'équation ci-dessous :

$$CL = -10.3 \cdot CorrP - 9.65 \cdot ContC\_v2 + 12.$$

De plus, pour chaque valeur des paramètres de corrélation CorrP et de contraste ContC, l'équation P ci-dessous permet de déterminer une probabilité de présence d'un élément de croissance tel que des colonies de micro-organismes à partir de la valeur de CL définie ci-dessus.

$$P = \frac{1}{1 + \exp(-CL)}$$

Afin de classifier les zones potentielles de croissance l'utilisateur peut fixer un critère de décision sur les valeurs de P obtenues.

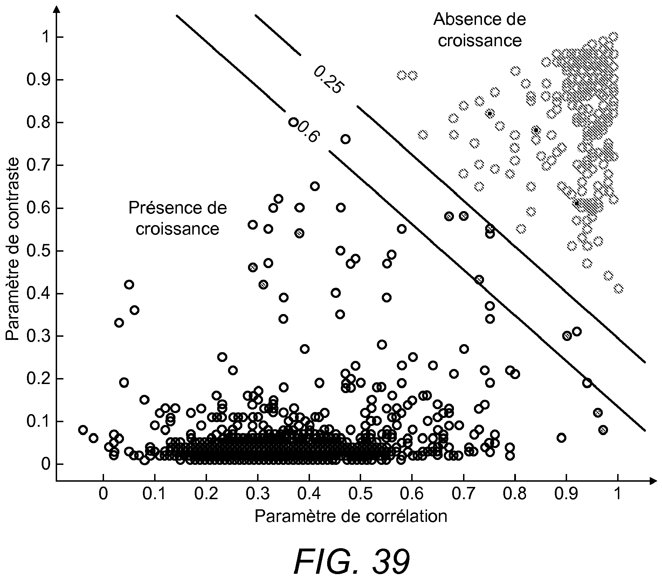

Selon un mode de réalisation de la présente invention montré sur la figure 39, le critère de décision P est fixé avec les conditions ci-dessous :

- si P<0,25 alors la zone potentielle de croissance correspond à un élément stable dans le temps donc la zone potentielle de croissance n'est pas une zone de croissance, il s'agit d'une zone dite négative ;
- si P>0,6 alors la zone potentielle de croissance correspond à un élément qui évolue au cours du temps et donc la zone potentielle de croissance est une zone de croissance avérée, il s'agit d'une zone dite positive ;
- si 0,25<P<0.6 alors il n'est pas possible de déterminer si la zone potentielle de croissance correspond à un élément stable ou à un élément qui évolue au cours du temps, c'est-à-dire que la distinction entre une zone dite positive et une zone dite négative n'est pas possible.

Le procédé d'analyse temporelle permet ensuite de comparer dans une étape 474 la valeur de l'annotation locale avec le critère de décision P, donc de rechercher, pour chaque zone potentielle de croissance d'une image finale, la correspondance avec l'image initiale correspondante. Ainsi dans une étape 476, l'utilisateur peut déterminer l'absence ou la présence de colonies de micro-organismes au sein de la boîte de Petri analysée.

Après le calcul des paramètres de corrélation et de contraste, le procédé d'analyse temporelle permet de déterminer si la zone de croissance correspond à un élément ayant évolué ou n'ayant pas évolué au cours du temps.

Lorsque la zone de croissance correspond à un élément ayant évolué au cours du temps, le procédé d'analyse temporelle permet de confirmer la validité de la zone de croissance telle que déterminée par le procédé de détermination préalable de croissance. Ainsi, la zone de croissance correspond effectivement à une croissance de micro-organismes.

Lorsque la zone de croissance correspond à un élément n'ayant pas évolué au cours du temps, tel qu'un défaut lié au matériau de la boîte de Petri, le procédé d'analyse temporelle permet d'obtenir une correction de la zone de croissance. Ainsi, la zone de croissance corrigée indique que l'élément observé au sein de ladite zone de croissance ne correspond pas à une croissance de micro-organismes.

Après la détermination de la croissance, l'utilisateur a la possibilité d'identifier la nature des colonies de micro-organismes au moyen d'un système d'identification (non montré).

## Revendications

1. Procédé de détermination de la croissance de micro-organismes dans un échantillon biologique susceptible de contenir des micro-organismes, ledit échantillon biologique étant contenu dans un récipient d'analyse tel qu'une boîte de Petri, ledit récipient d'analyse étant soumis à une incubation d'une durée déterminée, ledit procédé comprenant les étapes suivantes :

   - acquisition d'une première pluralité d'images initiales du récipient d'analyse à un premier temps d'acquisition T1, avant ou pendant l'incubation ;
   - acquisition d'une deuxième pluralité d'images finales du récipient d'analyse à un deuxième temps d'acquisition T2, pendant ou après l'incubation ;
   - recalage de chaque image initiale de la première pluralité d'images initiales acquises, avec chaque image finale correspondante de la deuxième pluralité d'images finales acquises ;
   - localisation d'au moins une zone potentielle de croissance de micro-organismes au sein d'au moins une image de la deuxième pluralité d'images acquises ;
   - évaluation du contenu de la zone potentielle de croissance de micro-organismes identifiée afin de déterminer la présence de micro-organismes,

   dans lequel l'étape de recalage comprend une étape de recalage secondaire associée au contenu du récipient d'analyse, l'étape de recalage secondaire comprenant la création d'un maillage de sous-images pour chaque image initiale et chaque image finale.

2. Procédé de détermination de la croissance de micro-organismes selon la revendication 1, dans lequel l'étape de recalage secondaire comprend une étape de recalage local de chaque couple de sous-images.

**3.** Procédé de détermination de la croissance de micro-organismes selon la revendication 2, dans lequel l'étape de recalage secondaire comprend une étape de recalage global dont les paramètres de recalage global sont déduits, pour chaque couple de sous-images, de ladite étape de recalage local.

**4.** Procédé de détermination de la croissance de micro-organismes selon l'une des revendications précédentes, l'étape de recalage comprenant une étape de recalage primaire associée à un identifiant localisé sur le récipient d'analyse.

**5.** Procédé de détermination de la croissance de micro-organismes selon la revendication 4, dans lequel le récipient d'analyse est une boîte de Petri, l'identifiant est une étiquette latérale disposée sur la paroi latérale du réceptacle de ladite boîte de Petri, et dans lequel l'étape de recalage primaire comprend une étape de détection de l'étiquette latérale et un procédé de détermination des paramètres de recalage primaire associés aux caractéristiques de l'étiquette latérale.

**6.** Procédé de détermination de la croissance de micro-organismes selon la revendication 5, dans lequel l'étape de détection de l'étiquette latérale comprend une première étape de détection des bords de la boîte de Petri et une deuxième étape de localisation de l'étiquette latérale.

**7.** Procédé de détermination de la croissance de micro-organismes selon l'une des revendications précédentes, l'étape de localisation d'au moins une zone potentielle de croissance de micro-organismes comprenant une étape de détection d'une densité élevée de colonies de micro-organismes, une étape de détection de colonies de micro-organismes non-circulaires et une étape d'attribution d'un élément de localisation à ladite zone potentielle de croissance de micro-organismes.

**8.** Procédé de détermination de la croissance de micro-organismes selon l'une des revendications précédentes, l'étape d'évaluation du contenu de la zone potentielle de croissance identifiée comprenant une étape de détermination des valeurs de deux paramètres d'évolution.

**9.** Procédé de détermination de la croissance de micro-organismes selon la revendication 8, les deux paramètres d'évolution comprenant un paramètre de corrélation et un paramètre de contraste.

**10.** Système de détermination de la croissance de micro-organismes dans un échantillon biologique susceptible de contenir des micro-organismes, ledit échantillon biologique étant contenu dans un récipient d'analyse telle qu'une boîte de Petri, ledit récipient d'analyse étant soumis à une incubation d'une durée déterminée, ledit système comprenant :

- un dispositif de capture d'images (12) afin d'acquérir une pluralité d'images de l'objet à analyser à un premier temps d'acquisition T1, avant ou pendant l'incubation, et à un deuxième temps d'acquisition T2, pendant ou après l'incubation ;
- un dispositif d'illumination (14) comprenant une ou plusieurs sources lumineuses afin d'illuminer le récipient d'analyse ;
- un dispositif de contrôle (18) pour contrôler l'application du procédé de détermination de la croissance de micro-organismes selon l'une des revendications 1 à 9 afin de déterminer la présence de micro-organismes.

**11.** Produit programme d'ordinateur comprenant des instructions logicielles pour la mise en oeuvre d'un procédé selon l'une des revendications 1 à 9 lorsque ledit programme est exécuté par un processeur de données.

**Patentansprüche**

**1.** Ein Verfahren zur Bestimmung des Wachstums von Mikroorganismen in einer biologischen Probe, die voraussichtlich Mikroorganismen enthält, wobei die biologische Probe in einem Analysebehälter wie etwa einer Petrischale enthalten ist, wobei der Analysebehälter für eine bestimmte Zeit einer Inkubation unterzogen wird, wobei das Verfahren die folgenden Schritte beinhaltet:

- Erfassen einer ersten Vielzahl von anfänglichen Bildern des Analysebehälters zu einem ersten Erfassungszeitpunkt T1 vor oder während der Inkubation;
- Erfassen einer zweiten Vielzahl von endgültigen Bildern des Analysebehälters zu einem zweiten Erfassungszeitpunkt T2 während oder nach der Inkubation;

- Registrieren jedes anfänglichen Bilds der ersten Vielzahl von erfassten anfänglichen Bildern mit jedem entsprechenden endgültigen Bild der zweiten Vielzahl von erfassten endgültigen Bildern;
- Lokalisieren von mindestens einer potenziellen Mikroorganismen-Wachstumszone innerhalb mindestens eines Bilds der zweiten Vielzahl von erfassten Bildern;
- Beurteilen des Inhalts der identifizierten potenziellen Mikroorganismen-Wachstumszone zum Bestimmen des Vorhandenseins von Mikroorganismen,

wobei der Registrierungsschritt einen sekundären Registrierungsschritt beinhaltet, der mit dem Inhalt des Analysebehälters assoziiert ist, wobei der sekundäre Registrierungsschritt das Erzeugen eines Netzes von Unterbildern für jedes anfängliche Bild und jedes endgültige Bild beinhaltet.

2. Verfahren zur Bestimmung des Wachstums von Mikroorganismen gemäß Anspruch 1, wobei der sekundäre Registrierungsschritt einen lokalen Registrierungsschritt jedes Paares von Unterbildern beinhaltet.

3. Verfahren zur Bestimmung des Wachstums von Mikroorganismen gemäß Anspruch 2, wobei der sekundäre Registrierungsschritt einen globalen Registrierungsschritt beinhaltet, für den die globalen Registrierungsparameter für jedes Paar von Unterbildern von dem lokalen Registrierungsschritt abgeleitet werden.

4. Verfahren zur Bestimmung des Wachstums von Mikroorganismen gemäß einem der vorhergehenden Ansprüche, wobei der Registrierungsschritt einen primären Registrierungsschritt beinhaltet, der mit einem Identifikator assoziiert ist, der auf dem Analysebehälter lokalisiert ist.

5. Verfahren zur Bestimmung des Wachstums von Mikroorganismen gemäß Anspruch 4, wobei der Analysebehälter eine Petrischale ist, der Identifikator ein an der Seitenwand des Behälters der Petrischale angeordnetes Seitenetikett ist, und wobei der primäre Registrierungsschritt einen Seitenetikettendetektionsschritt und ein Verfahren zur Bestimmung der primären Registrierungsparameter, die mit den Charakteristiken des Seitenetiketts assoziiert sind, beinhaltet.

6. Verfahren zur Bestimmung des Wachstums von Mikroorganismen gemäß Anspruch 5, wobei der Seitenetikettendetektionsschritt einen ersten Schritt des Detektierens der Ränder der Petrischale und einen zweiten Schritt des Lokalisierens des Seitenetiketts beinhaltet.

7. Verfahren zur Bestimmung des Wachstums von Mikroorganismen gemäß einem der vorhergehenden Ansprüche, wobei der Schritt des Lokalisierens mindestens einer potenziellen Mikroorganismen-Wachstumszone einen Schritt des Detektierens einer hohen Dichte von Mikroorganismenkolonien, einen Schritt des Detektierens nichtzirkulärer Mikroorganismenkolonien und einen Schritt des Zuweisens eines Lokalisierungselements an die potentielle Mikroorganismen-Wachstumszone beinhaltet.

8. Verfahren zur Bestimmung des Wachstums von Mikroorganismen gemäß einem der vorhergehenden Ansprüche, wobei der Schritt des Beurteilens des Inhalts der identifizierten potenziellen Wachstumszone einen Schritt der Bestimmung der Werte von zwei Entwicklungsparametern beinhaltet.

9. Verfahren zur Bestimmung des Wachstums von Mikroorganismen gemäß Anspruch 8, wobei die zwei Entwicklungsparameter einen Korrelationsparameter und einen Kontrastparameter beinhalten.

10. Ein System zur Bestimmung des Wachstums von Mikroorganismen in einer biologischen Probe, die voraussichtlich Mikroorganismen enthält, wobei die biologische Probe in einem Analysebehälter wie etwa einer Petrischale enthalten ist, wobei der Analysebehälter für eine bestimmte Zeit einer Inkubation unterzogen wird, wobei das System Folgendes beinhaltet:

- eine Bildfesthaltungsvorrichtung (12) zum Erfassen einer Vielzahl von Bildern des zu analysierenden Objekts zu einem ersten Erfassungszeitpunkt T1 vor oder während der Inkubation und zu einem zweiten Erfassungszeitpunkt T2 während oder nach der Inkubation;
- eine Beleuchtungsvorrichtung (14), beinhaltend eine oder mehrere Lichtquellen zum Beleuchten des Analysebehälters;
- eine Steuerungsvorrichtung (18) zur Steuerung der Anwendung des Verfahrens zur Bestimmung des Wachstums von Mikroorganismen gemäß einem der Ansprüche 1 bis 9 zur Bestimmung der Anwesenheit von Mikroorganismen.

**11.** Ein Computerprogrammprodukt, beinhaltend Softwareanweisungen zur Implementierung eines Verfahrens gemäß einem der Ansprüche 1 bis 9, wenn das Programm von einem Datenprozessor ausgeführt wird.

**Claims**

**1.** A method for determining microorganism growth in a biological sample likely to contain microorganisms, said biological sample being contained in an analysis container such as a Petri dish, said analysis container being subjected to an incubation of a determined duration, said method comprising the following steps:

- acquisition of a first plurality of initial images of the analysis container at a first acquisition time T1, before or during the incubation;
- acquisition of a second plurality of final images of the analysis container at a second acquisition time T2, during or after the incubation;
- registration of each initial image of the first plurality of initial images acquired, with each corresponding final image of the second plurality of final images acquired;
- location of at least one potential microorganism growth zone in at least one image of the second plurality of images acquired;
- evaluation of the content of the potential microorganism growth zone identified in order to determine the presence of microorganisms,

in which the registration step comprises a secondary registration step associated with the content of the analysis container, the secondary registration step comprising the creation of a grid of subimages for each initial image and each final image.

**2.** The method for determining microorganism growth according to Claim 1, in which the secondary registration step comprises a step of local registration of each pair of subimages.

**3.** The method for determining microorganism growth according to Claim 2, in which the secondary registration step comprises a step of global registration for which the global registration parameters are deduced, for each pair of subimages, from said local registration step.

**4.** The method for determining microorganism growth according to one of the preceding claims, the registration step comprising a primary registration step associated with an identifier located on the analysis container.

**5.** The method for determining microorganism growth according to Claim 4, in which the analysis container is a Petri dish, the identifier is a lateral label arranged on the lateral wall of the receptacle of said Petri dish, and in which the primary registration step comprises a step of detection of the lateral label and a method for determining primary registration parameters associated with the characteristics of the lateral label.

**6.** The method for determining microorganism growth according to Claim 5, in which the step of detection of the lateral label comprises a first step of detection of the edges of the Petri dish and a second step of location of the lateral label.

**7.** The method for determining microorganism growth according to one of the preceding claims, the step of location of at least one potential microorganism growth zone comprising a step of detection of a high density of microorganism colonies, a step of detection of non-circular microorganism colonies and a step of assignment of a location element to said potential microorganism growth zone.

**8.** The method for determining microorganism growth according to one of the preceding claims, the step of evaluation of the content of the potential growth zone identified comprising a step of determination of the values of two parameters of change.

**9.** The method for determining microorganism growth according to Claim 8, the two parameters of change comprising a correlation parameter and a contrast parameter.

**10.** A system for determining microorganism growth in a biological sample likely to contain microorganisms, said biological sample being contained in an analysis container such as a Petri dish, said analysis container being subjected to an incubation of a determined duration, said system comprising:

- an image capture device (12) in order to acquire a plurality of images of the object to be analysed at a first acquisition time T1, before or during the incubation, and at a second acquisition time T2, during or after the incubation;
- an illumination device (14) comprising one or more light sources in order to illuminate the analysis container;
- a control device (18) for controlling the application of the method for determining microorganism growth according to one of Claims 1 to 9 in order to determine the presence of microorganisms.

11. A computer program product comprising software instructions for implementing a method according to one of Claims 1 to 9 when said program is run by a data processor.

10 ~

| | 12 |
|---|---|
| Dispositif de capture d'images | |

| | 14 |
|---|---|
| Dispositif d'illumination | |

| | 16 |
|---|---|
| Dispositif d'analyse d'images | |

| | 18 |
|---|---|
| Dispositif de contrôle | |

| | 20 |
|---|---|
| Dispositif d'incubation | |

## FIG. 1

| | 22 |
|---|---|
| Acquisition d'une première série d'images I1 à T=T1 | |

| | 24 |
|---|---|
| Acquisition d'une deuxième série d'images I2 à T=T2 | |

| | 26 |
|---|---|
| Recalage primaire | |

| | 28 |
|---|---|
| Recalage secondaire | |

| | 30 |
|---|---|
| Détermination préalable de croissance de micro-organismes | |

| | 40 |
|---|---|
| Analyse temporelle | |

## FIG. 2

1001 →

```
┌──────────────────────┐  ⌐~100
│   Détection de la     │
│   boite de Petri      │
└──────────────────────┘
           │
┌──────────────────────┐  ⌐~110
│   Localisation de     │
│  l'étiquette latérale │
└──────────────────────┘
           │
┌──────────────────────┐  ⌐~120
│   Suppression des     │
│      défauts          │
│   d'illumination      │
└──────────────────────┘
           │
┌──────────────────────┐  ⌐~130
│   Détermination de    │
│  l'étiquette latérale │
│      théorique        │
└──────────────────────┘
```

1002 →

```
┌──────────────────────┐  ⌐~140
│   Détermination des   │
│   orientations des    │
│  étiquettes latérales │
│   théoriques et d'un  │
│     intervalle de     │
│  valeurs des angles   │
│     de rotation       │
└──────────────────────┘
           │
┌──────────────────────┐  ⌐~150
│   Recalage optimal    │
│                       │
└──────────────────────┘
```

## FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

## FIG. 16

## FIG. 17

**FIG. 18**

**FIG. 19**

FIG. 20

FIG. 21

*FIG. 22*

*FIG. 23*

*FIG. 24*

*FIG. 25*

*270*

272 — Calcul de la valeur d'un
déplacement optimal

273 — Calcul de la valeur du
paramètre de similarité

274 — Calcul de la valeur d'un
paramètre de contraste

## FIG. 26

FIG. 27

FIG. 28

FIG. 29

```
┌─────────────────────────────────┐
│      Obtention d'images          │ ～300
└─────────────────────────────────┘
                │
┌─────────────────────────────────┐
│   Transformation des images      │ ～302
└─────────────────────────────────┘
                │
┌─────────────────────────────────┐
│   Détection d'une densité élevée │ ～304
│   de colonies et de colonies non-│
│            circulaires           │
└─────────────────────────────────┘
                │
┌─────────────────────────────────┐
│    Association d'un rectangle    │ ～306
│   englobant à chaque zone de     │
│      croissance détectée         │
└─────────────────────────────────┘
```

## FIG. 30

```
┌─────────────────────────────────┐
│   Prétraitement des images       │ ～420
└─────────────────────────────────┘
                │
┌─────────────────────────────────┐
│   Evaluation des paramètres      │ ～430
│         d'évolution              │
└─────────────────────────────────┘
                │
┌─────────────────────────────────┐
│  Détermination de la croissance  │ ～440
│            effective             │
└─────────────────────────────────┘
```

## FIG. 31

420

Amélioration du contraste — 424

Réduction du bruit — 425

## FIG. 32

| | Backlight | Bottom | Median |
|---|---|---|---|
| Sous-image initiale (à T1) | | | |
| Sous-image finale (à T2) | | | |

**FIG. 33**

| | Backlight | Bottom | Median |
|---|---|---|---|
| Sous-image initiale (à T1) | | | |
| Sous-image finale (à T2) | | | |

**FIG. 34**

430

Evaluation du premier
paramètre d'évolution — 461

Evaluation du deuxième
paramètre d'évolution — 462

## FIG. 35

461

Mesure du contraste pour
chaque image initiale et chaque
condition d'acquisition ⌐463

Mesure du contraste pour
chaque image finale et chaque
condition d'acquisition ⌐464

Calcul du ratio de la mesure du
contraste par couple d'images ⌐465

Détermination de la valeur du
paramètre de contraste ⌐466

## FIG. 36

*462*

Sélection de la sous-image
initiale en couleur la plus
contrastée ~450

Sélection de la sous-image
finale prétraitée la plus
contrastée en niveaux de gris ~451

Transformation des
sous-images colorées en
images en niveaux de gris ~452

Calcul du coefficient de
corrélation ~453

Calcul du paramètre de
corrélation ~454

*FIG. 37*

**440**

| Listage des paramètres d'évolution | ~470 |
| Application d'une méthode de régression | ~472 |
| Comparaison avec le critère de décision | ~474 |
| Détermination de la croissance effective | ~476 |

*FIG. 38*

FIG. 39

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0796319 A
- US 5510246 A
- WO 2014059357 A
- EP 1061127 A
- WO 03022999 A
- WO 2012152769 A

**Littérature non-brevet citée dans la description**

- **DEN HERTOG, A. et al.** Simplified Automated Image Analysis for Détection and Phenotyping of Mycobacterium tubercolisis on Porous Supports by Monitoring Growing Microcolonies. *PLos ONE,* 2010, vol. 5 (6), e11008
- **CHEN W-B.** An automated bacterial colony counting and classification system. *Springer Science + Business Media,* 2009, vol. 11, 349-368